# FASCICULE DE BREVET EUROPEEN

(11) **EP 0 788 782 B1**
(45) Date de publication et mention de la délivrance du brevet: **05.12.2001**
(21) Numéro de dépôt: 97400189.3
(22) Date de dépôt: 28.01.1997
(51) Int. Cl.: A61F 2/30, A61F 2/36

(54) **Prothèse articulaire assemblée par gradins**
Durch Stufen verbundene Gelenkprothese
Joint prosthesis connected by steps

(30) Priorité: 08.02.1996 FR 9601539
(43) Date de publication de la demande: 13.08.1997
(73) Titulaire: Deckner, André Georges, F-75015 Paris (FR)
(72) Inventeur: Deckner, André Georges, F-75015 Paris (FR)
(74) Mandataire: Eidelsberg, Victor Albert

(56) Documents cités:
- EP-A- 0 119 321
- EP-A- 0 308 297
- EP-A- 0 681 816
- WO-A-95/13757
- DE-A- 4 441 178
- US-A- 5 405 392

## Description

L'invention se rapporte aux implants chirurgicaux, notamment prothèses articulaires destinées notamment aux prothèses de hanche.

On connaît déjà une prothèse articulaire comprenant une pièce proximale et une pièce distale. Ces pièces sont assemblées l'une à l'autre par une jonction morse. Toutes les jonctions connues donnent lieu à des micro-mouvements après un grand nombre de mises en charge Ces micro-mouvements apparaissent du fait de la trop grande différence des contraintes localisées dans les masses métalliques au voisinage immédiat de part et d'autre d'un point de la jonction, dit plus bas "point de jonction". Les jonctions usuelles présentent de très fortes variations de section, au voisinage de la jonction, dans les deux composants à assembler. Ces variations excessives de section et de forme ont pour conséquence de fortes variations de contraintes au sein des matériaux, au moment des mises en charge répétées. Il apparaît des micro-mouvements relatifs de part et d'autre dudit point de jonction. Ces micro-mouvements provoquent à la longue l'abrasion mutuelle des deux pièces, leur usure et donne des débris d'usure.

Les matériaux constituant les surfaces de jonction et constituant les composants à assembler peuvent être chacun métalliques, céramiques ou plastiques.

L'invention vise à réduire, de façon significative, les différences de contraintes dans les masses au voisinage du point de jonction et de retarder de façon importante l'apparition de l'usure. Bien que la pièce proximale et la pièce distale aient toujours chacune une partie affaiblie, la résistance mécanique de la jonction est améliorée en sorte qu'elle peut résister aux sollicitations provoquées par la marche du patient pendant plusieurs années.

L'invention a donc pour objet une prothèse articulaire comprenant une pièce proximale et une pièce distale de forme telle que l'une d'entre elles soit femelle et l'autre mâle lorsqu'elles sont emmanchées suivant un axe d'impaction par des faces en regard. Suivant l'invention, chaque face en regard comporte plusieurs tronçons en gradins, chaque tronçon étant tronconique, l'épaisseur des tronçons consécutifs de l'une des pièces étant croissante, alors que l'épaisseur des tronçons consécutifs de l'autre pièce est décroissante.

L'épaisseur est définie par une dimension suivant une direction perpendiculaire à l'axe d'impaction, qui se confond avec la direction d'emmanchage.

Grâce au fait que chaque face en regard est subdivisée en plusieurs tronçons d'épaisseurs calculées indépendamment, on peut donner à chacun de ces tronçons une conicité suffisamment petite (angle de la génératrice avec l'axe d'impaction), cet angle petit étant nécessaire pour assurer le bon accrochage des surfaces en regard par emmanchement conique. Bien que la longueur de la région d'assemblage soit dans une prothèse articulaire nécessairement relativement petite, la succession des tronçons permet un transfert progressif d'un composant à l'autre de la charge appliquée à la jonction, en sorte que les parties affaiblies, parce qu'amincies, ne supportent plus seules des charges trop grandes susceptibles d'en provoquer la rupture. On prévoit des gradins car, contrairement à une jonction à cône unique de même angle, les gradins n'entraînent pas pour l'une ou les deux pièces une faiblesse mécanique inacceptable, et de brutales variations d'épaisseur dans les matériaux, les contraintes excessives se localisant justement dans le voisinage de brutales variations de section transversale.

Le brevet U.S. n° 5 405 392 ne décrit pas que l'épaisseur des tronçons consécutifs de l'une des pièces est croissante, alors que l'épaisseur des tronçons consécutifs de l'autre pièce est décroissante. Grâce à cela, on diminue les différences de contraintes dans les masses au voisinage du point de jonction et on retarde l'apparition de l'usure. Ce résultat n'est pas non plus décrit au brevet américain.

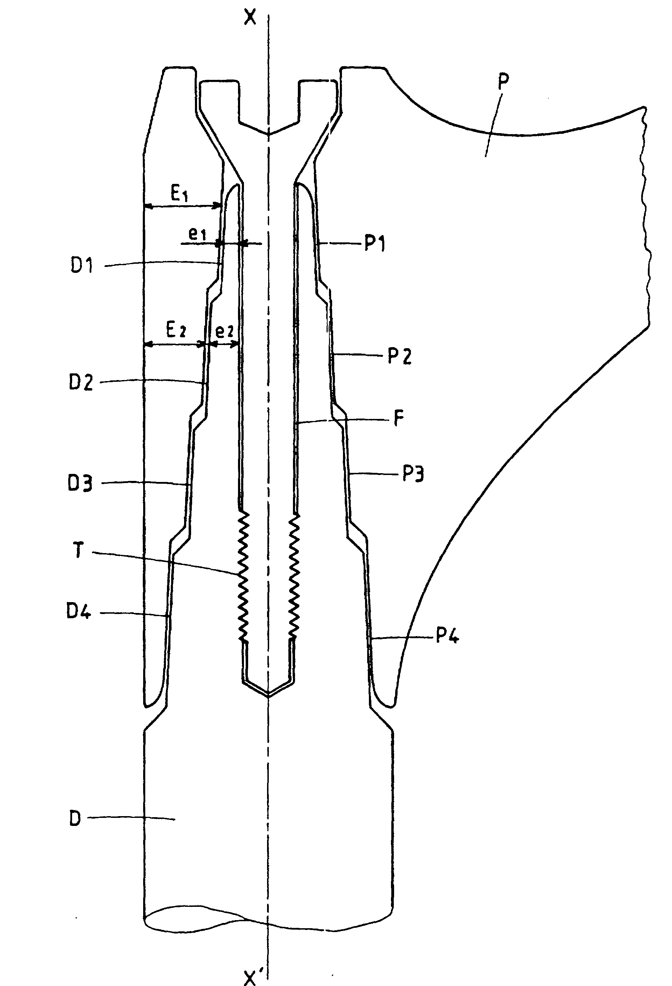

A la figure 12 du EP-A-0 308 297 on décrit une pièce acétabulaire pour prothèse ayant une coquille délimitant un logement tronconique à épaulement dans lequel s'emboîte à force une cupule 42. On ne précise pas la forme de la cupule. Les sections transversales tant de la cupule que de la coquille sont croissantes dans le même sens d'un côté de l'épaulement à l'autre.

Chaque pièce comporte un petit nombre (de 2 à 20) de gradins, et l'étendue axiale de chaque gradin est supérieure à 1 mm.

Les gradins ne constituent pas eux-mêmes une microstructure de surface ou un système d'accrochage comportant des dents, mais travaillent chacun comme des jonctions coniques en étant associés à leur propre tronçon conjugué.

De préférence, l'angle de la génératrice de chaque tronçon avec l'axe xx' d'impaction est inférieur à 5° et de préférence à 3°.

Suivant un perfectionnement, les deux pièces à assembler sont, non seulement impactées dans la direction de l'axe, mais sont en plus mises en forte traction l'une en direction de l'autre le long de l'axe, de sorte que la pièce mâle se trouve légèrement comprimée et que la pièce femelle soit légèrement dilatée dans la limite des propriétés élastiques propres à chaque matériau Cela constitue une mise en précontrainte de la jonction et augmente la limite à partir de laquelle des micro-mouvements relatifs apparaissent au cours du fonctionnement.

Cette précontrainte peut être obtenue par exemple par une extrémité filetée de la partie mâle sur laquelle est serré un écrou s'appuyant sur la partie femelle, ou par exemple par une tige filetée serrant la partie femelle et prenant appui dans un trou taraudé dans la partie mâle.

Les conicités de tronçons successifs de l'une des pièces peuvent être différentes, dans le but de réaliser volontairement une meilleure répartition des contraintes le long de la jonction pour s'adapter aux différentes épaisseurs de matériau.

Suivant un perfectionnement, un tronçon de l'une des pièces conjugué d'un tronçon de l'autre pièce a une conicité différente de celle du tronçon conjugué pour procurer une meilleure répartition des contraintes entre les deux extrémités du même tronçon.

La figure unique du dessin annexé illustre l'invention.

La figure est une vue en coupe d'une prothèse articulaire suivant l'invention. Cette prothèse comporte une pièce proximale P femelle et une pièce distale D, mâle pénétrant dans la pièce proximale P. La pièce P comporte à cet effet un alésage central ayant quatre tronçons en gradins P1, P2, P3, P4, l'épaisseur E2 du gradin P2 étant plus petite que celle E1 du gradin P1 et ainsi de suite en fonction des gradins.

La pièce D distale comporte, elle aussi, quatre gradins D1, D2, D3, D4 conjugués des gradins P1, P2, P3, P4 L'épaisseur e1 du gradin D1 étant cette fois plus petite que l'épaisseur e2 du gradin D2 et ainsi de suite pour les gradins D3 et D4. Chaque tronçon P1, P4 et son conjugué D1, D4 sont tronconiques en faisant avec l'axe xx' d'impaction un angle voisin de 4°.

La pièce distale D comporte un taraudage T de même axe que l'axe xx' d'impaction dans lequel est vissée une tige filetée F de précontrainte axiale.

## Revendications

1. Prothèse articulaire comprenant une pièce (P) proximale et une pièce (D) distale de forme telle que l'une d'entré elles soit femelle et l'autre mâle lorsqu'elles sont emmanchées suivant un axe (XX') d'impaction par des faces en regard conjuguées, chaque face en regard comportant plusieurs tronçons en gradins, chaque tronçon étant tronconique, **caractérisée en ce que**, dans une section suivant une vue en coupe dans un plan passant par l'axe (XX') d'impaction, une épaisseur (E1, E2), dans une direction perpendiculaire à l'axe (XX') d'impaction, de tous les tronçons consécutifs de l'une des pièces est croissante, alors qu'une épaisseur (e1, e2), dans la même direction perpendiculaire à l'axe (XX') d'impaction, de tous les tronçons consécutifs conjugués de l'autre pièce est décroissante.

2. Prothèse suivant la revendication 1, **caractérisée en ce que** l'angle de la génératrice de chaque tronçon avec l'axe (XX') d'impaction est inférieur à 5° et de préférence à 3°.

3. Prothèse suivant la revendication 1 ou 2, **caractérisée par** des moyens (F) de précontrainte axiale des pièces (P, D).

4. Prothèse suivant la revendication 3, **caractérisée en ce que** l'une des deux pièces (P, D) comporte un taraudage (T) de même axe que l'axe (XX') d'impaction dans lequel est vissée une tige filetée (F) de précontrainte axiale.

5. Prothèse suivant l'une des revendications précédentes, **caractérisée en ce que** les conicités de tronçons successifs de l'une des pièces (P, D) sont différentes.

6. Prothèse suivant l'une des revendications précédentes, **caractérisée en ce qu'**un tronçon de l'une des pièces (P ou D) conjugué d'un tronçon de l'autre pièce (D ou P) a une conicité différente de celle du tronçon conjugué.

## Patentansprüche

1. Gelenkprothese, die ein proximales Teil (P) und ein distales Teil (D) dergestalt beinhaltet, dass eines von beiden weiblich und das andere männlich ist, wenn sie entlang einer Wirkungsachse (XX') durch sich gegenüberliegende paarige Seiten eingepresst sind, wobei jede der gegenüberliegenden Seiten mehrere stufige Abschnitte beinhaltet, die jeweils kegelstumpfartig sind,
**dadurch gekennzeichnet, dass** in einem Abschnitt gemäß einer durch die Wirkungsachse verlaufenden Schnittfläche in einer zu der Wirkungsachse (XX') senkrechten Richtung eine Dicke (E1, E2) aller konsekutiven Abschnitte von einem der Teile ansteigend ist, während in der selben senkrechten Richtung zu der Wirkungsachse (XX') eine Dicke (e1, e2) aller konsekutiven paarigen Abschnitte des anderen Teils abnehmend ist.

2. Prothese gemäß Anspruch 1,
**dadurch gekennzeichnet, dass** der Kantenwinkel jedes Abschnitts zu der Wirkungsachse (XX') kleiner als 5° und vorzugsweise kleiner als 3° ist.

3. Prothese gemäß Anspruch 1 oder 2,
**gekennzeichnet durch** Mittel (F) zur axialen Vorspannung der Teile (P, D).

4. Prothese gemäß Anspruch 3,
**dadurch gekennzeichnet, dass** eines der beiden Teile (P, D) eine Gewindebohrung (T) in der gleichen Achse wie die Wirkungsachse (XX') beinhaltet, in welcher eine Gewindestange (F) zur axialen Vorspannung festgeschraubt ist.

5. Prothese gemäß einem der vorangegangenen Ansprüche,
**dadurch gekennzeichnet, dass** die Kegelgrößen der aufeinanderfolgenden Abschnitte von,einem der Teile (P, D) unterschiedlich sind.

6. Prothese gemäß einem der vorangegangenen Ansprüche,
**dadurch gekennzeichnet, dass** ein Abschnitt von einem der Teile (P, D), der mit einem Abschnitt des anderen Teils (D oder P) zusammenwirkt, eine vom paarigen Abschnitt unterschiedliche Kegelgröße aufweist.

## Claims

1. Articular prosthesis comprising a proximal (P) part and a distal part (D) having a form such that one part is female and the other part is male when said parts are assembled along an impaction axis (XX') with respective mating faces opposite each other, each opposite face comprising several stepped sections, each section being in the form of a truncated cone, **characterized in that**, in sectional view through a plan comprising said impaction axis (XX'), a thickness (E1, E2), in a direction perpendicularly to said axis (XX'), of all consecutives sections of one of the parts is increasing, while a thickness (e1, e2) in the same direction perpendicularly to said axis (XX') of all the mating consecutive sections of the other part is decreasing.

2. Prosthesis according to claim 1, **characterized in that** the angle between generator of each section and the impaction axis (XX') is less than 5°, and preferably less than 3°.

3. Prosthesis according to claim 1 or 2, **characterized by** means (F) for axially prestressing the parts (P, D).

4. Prosthesis according to claim 3, **characterized in that** one of the two parts (P, D) comprises a tapping (T) having an axis coincident with the impaction axis (XX'), into which is screwed a threaded rod (F) which has been axially prestressed.

5. Prosthesis accord to one of claims 1 to 4, **characterized in that** the successive sections of one of the parts (P, D) have different tapers.

6. Prosthesis according to one of claims 1 to 5, **characterized in that** one section of one of the parts (P or D) mating with a section of the other part (D or P) has a taper different from the one of the mating part.
